# EUROPEAN PATENT APPLICATION

(11) **EP 2 269 548 A1**
(43) Date of publication of application: **05.01.2011**
(21) Application number: 10164818.6
(22) Date of filing: 03.06.2010
(51) Int. Cl.: A61F 13/02, A61M 25/02, A61M 16/04

(54) **Preservation of exit site of an orthopedic pin or tube extruding from the skin**

(30) Priority: 04.06.2009 IL 19915909
(71) Applicant: Porat, Michael, Tel Aviv 69690 (IL)
(72) Inventor: Porat, Michael, Tel Aviv 69690 (IL)
(74) Representative: Perkins, Sarah

(57) **Abstract**

Use of two "U-Shaped" or "V-Shaped" or slit plasters/adhesive tape to be placed opposite one another over a pad placed around the extruding object from the skin to completely surround the extruding object and adhere the pad securely at the exit site.

The pad (2) is placed around the extruding object at the exit site (1). The adhesive tape (3) is adhered to affix the pad to the skin so that the extruding member reaches the bottom of the "U" or the "V" or the slit as closely as possible. The adhesive tape (4) is placed opposite the tape (3) to affix the rest of the pad to the skin, so that the extruding member touches the bottom of the "U" or the "V" or the slit of this adhesive as closely as possible.

## Description

The present invention relates mainly to the adhesion of a wound dressing around the exit site of an orthopedic pin or tube extruding from the skin. External fixation pins are widely used following orthopedic treatment of broken or treated bones. The extruding pin ends are held in place by a "bridge" that joins one pin to another to secure their position until the bones join and heal. At exit sites where pins or tubes extrude, localized skin eruptions can cause a wide range of potential infections that can deteriorate and in some cases, become life threatening. The use of antibiotics and gels has been the accepted method to prevent exacerbated infection at these exit sites.

Because the extruding pin has a direct connection from the "out side" into the body, the rate of contamination is very high. In medical practice this type of contamination is referred to as Pin Tract Infection (PTI). Researches reveal that despite constant twice daily cleansing of the exit-site by means of antiseptic solutions or antibiotics infection rates remain very high, and in one published research it reached 96.8% of treated patients.

The position of the "bridge" interferes with the positioning and fixation of a dressing around the exit site. The "bridge" above the pin, or its length in cases of catheter tubing, does not facilitate fixation of a regular pad. Pads with a center hole or pads with a slit at their center are well known in the art, and this facilitates placement around the extruding object. But, still, there is the problem of affixing the pad, as regular known systems of affixing them by means of bandages or adhesive tape do not provide complete medical efficacy. The pad and the skin should be completely surrounded and covered by the adhesive tape without any uneven wrinkles or gaps that will enable formation of a "bacterial tunnel" between the skin, the pad, and the adhesive material.

The following patents relate to suggested solutions or methods for the above-mentioned problem of preventing the PTI phenomena.

DE19830421 - Dressing for covering especially the edge areas of artificial body openings.

US5447492 - External fixation dressing for accommodating a retaining pin.

US2003018333 - Device and method to protect wounds and orthopedic pins from external contact.

US2003215307 - Pin cap.

US4856504 - Antimicrobial wound dressing and skin fixator for orthopedic pins.

US4915649 -Antimicrobial wound dressing and skin fixator for percutaneous conduits.

US20077293800 - Antimicrobial site dressings.

W09307928 -Medical apparatus fixation and infection control device.

US5360020 - Pin site shield retainer.

W02005009284 - Device for laceration or incision closure.

Antiseptic sponges, containing antisepsis or antibiotics, are used to protect and cure open wounds or skin exit sites. Over the past 10 years, the use of silver coated dressings - overcoming bacterial resistance - affixed to the skin has become a popular and effective method of infection and wound control for pressure wounds, burns, and at catheter and orthopedic pin exit sites. All of the above-mentioned antisepsis sponges, some of them also absorbent, are hereinafter called "pad" or "pads".

In order to keep the exit-site protected and clean, a pad should be placed around (360⁰) the extruding pin in order to completely surround it. For effective and successful treatment, pads must come into direct contact with the skin for best effectiveness during medical treatment.

The intention of our invention is to secure the pad around the skin exit site, by use of very pliable and soft adhesive tape that conforms to the body contour. If the adhesive tape is rigid, it will fall off quickly. A flexible adhesive tape with a central hole cannot effectively be placed to fit around the orthopedic pins due to interference of the "bridge" joining the pins. The same problem will be encountered if the tube is long and connected to a bag on one side.

The use of an adhesive with a centered hole and a slit towards the center can be introduced over the pin or tube through the slit and can fit around the pin. Disadvantage: Because of flexibility and high pliability of the exposed adhesive, it may be very difficult to position and affix accurately without any wrinkles.

Our suggested invention is the use of two "U-Shaped" or "V-Shaped" or slit plasters/adhesive tape to be placed opposite and overlapping one another around the extruding object to completely surround the pin and cover the skin securely at the exit site.

The pad is placed around the extruding member at the exit site. The U or V shaped or slit adhesive tape is adhered to affix the pad to the skin so that the extruding member closely reaches the bottom of the U or the V or the slits

The second U or V shaped or slit adhesive tape is then placed opposite the former tape overlapping the former tape to affix the rest of the pad to the skin, so that the extruding member touches the bottom of the U or the V or the slit of this adhesive as closely as possible.

This way the pad is in close touch with the skin around the exit site of the extruding member.

### DESCRIPTION OF THE FIGURES:

Figures 1 to 4, illustrates various shapes of pads (2). The pad can be rectangular or round with a central hole and a slit from the hole towards the edge of the pad. The pad can be rectangular or round with a cross-shaped slit at its center.
Figures 5 to 9 and Figures 10 to 14, illustrate various shapes of the adhesive tape (3) and (4) respectively, rectangular or half round, with a notch in a U or V shape or a slit (5) in one of the straight edges of the adhesive tape.

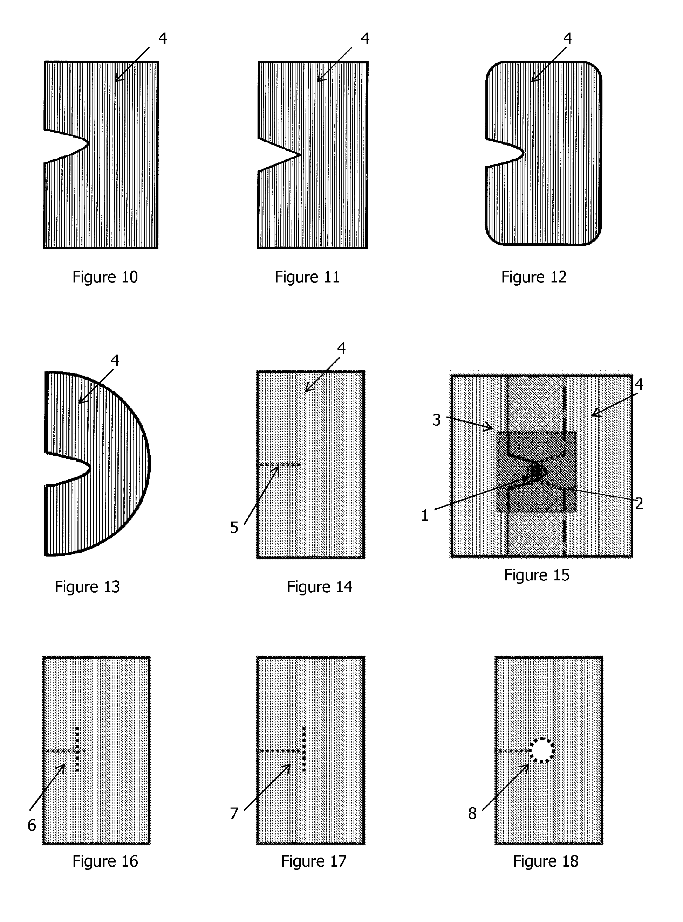

Figure 15 illustrates the final position of our invention. The pad (2) is placed around the extruding object at the exit site (1). The adhesive tape (3) is adhered to affix the pad to the skin so that the extruding member reaches the bottom of the U or the V or the slit as closely as possible. The adhesive tape (4) is placed opposite the tape (3) to affix the rest of the pad to the skin, so that the extruding member touches the bottom of the U or the V or the slit of this adhesive as closely as possible. It is needless to state that the pad can be also round or any other commonly used pad and the adhesive tape can be round or rectangular with lopped-off edges or a triangular (not shown in the figures) design.
Figure 16 shows a slit (6) in the adhesive tape in a cross shape.
Figure 17 shows a slit (7) in the adhesive tape in a T shape.
Figure 18 shows a slit (8) in the adhesive tape with a hole at the slit.

### DETAILED DESCRIPTION OF OUR PATENT:

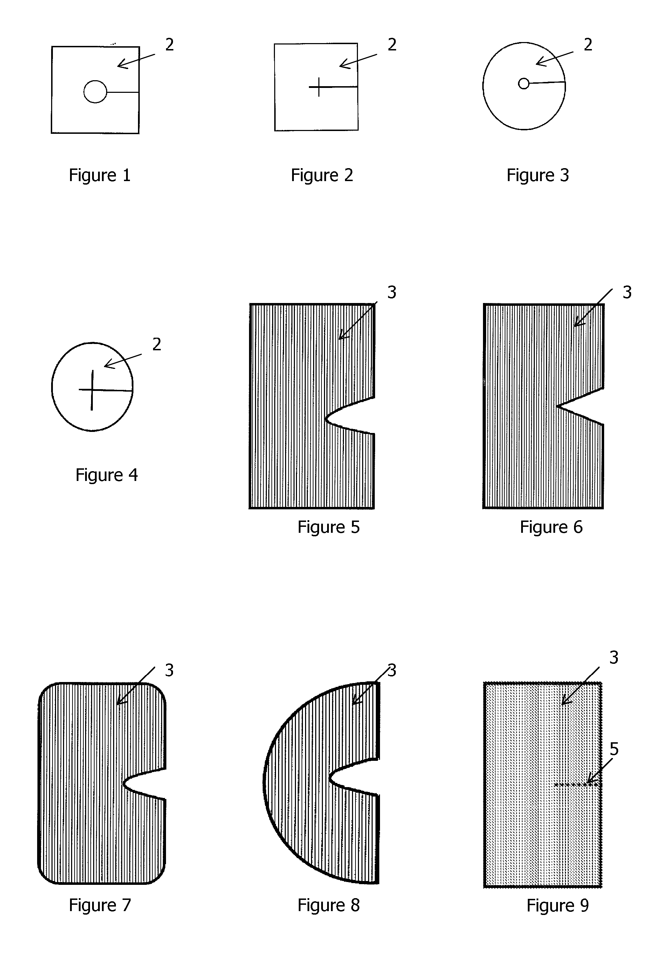

A pad containing antisepsis material (2 in all types of Figures 1 to 4), rectangular or round, with a slit towards its center, with or without a center hole, or a cross cut centered in the middle of the pad, placed around the pin.

Our suggested invention is the use of at least two "U-Shaped" or "V-Shaped" or slit plasters/adhesive tape (3 in all types of Figures 5 to 9 and 4 in all of Figures 10 to 14) to be placed around the pin (1 in Figure 15) opposite one another to completely surround the pin for a secure covering of the skin at the exit site.

The pad (2) is placed around the object extruding of the exit site (1 in Figure 15). The "U" or "V" shaped or slit adhesive tape (3) is adhered to affix the pad to the skin so that the extruding member reaches the bottom of the U or the V or the slit as closely as possible.

The "U" or "V" shaped or with the slit adhesive tape (4) is then placed opposite overlapping the tape (3) to affix the rest of the pad to the skin, so that the extruding member touches the bottom of the "U" or the "V" or the slit of this adhesive as closely as possible.

The adhesive tape can be rectangular, with or without lopped-off edges, round or triangular shaped. The notch in the adhesive tape can be "U" or "V" shaped with the wide part facing the adhesive tape edge. Instead of a notch, the adhesive tape can have a slit vertical to one of the edges. The slit can be straight, with or without a hole at its end or in a "T" or a cross shape.

The same purpose can be achieved by using one adhesive tape with a slit or a notch (similar to those shown in the figures); in this case, affixation is less convenient.

This way the pad is in close touch with the skin around the exit site of the extruded member.

In a better embodiment of our invention, the adhesive tape is porous and can be made of non-woven material, to allow permeation of air or liquids. It is suggested that the material used as a base for the adhesive be flexible and pliable so that the adhesion of the adhesive tape to the skin will not come apart due to movements of the skin onto which the tape is adhered. In our experiments we realized that a soft flexible material that is completely and flatly adhered to the skin, without wrinkles, will not detach from the skin even when wet, or after showering.

Medical Authorities allow certain pads containing silver to remain in contact with the skin for eight days whilst still being effective.

Current procedures for treatment of extruding pins, is to perform local on-site disinfection by means of antiseptic solution twice daily, (dressing removal, disinfection, and re-dressing). Our new invention will enable the effective use of the pad for a continuous period of eight days without removal. This is extremely efficient and saves staff time, and reduces patient discomfort.

Silver ions released from the silver pad have a greater influence on prevention of infection at the exit site if the pad is wet. It is therefore suggested that the absorbent silver pad be moistened with water during the period of protection at the exit site. The porous capability of the adhesive tape (known in the art) can be achieved by punching "holes" through the backing material and the adhesive. Another way to achieve the passage of liquid is by using the adhesive on the surface of a non-woven backing of adhesive tape in rows, (also known in the art) with no adhesive between the rows. This will improve the porosity of the adhesive tape and will enable easier and painless removal of the adhesive tape after use. All the attendant needs to do, is, by means of a syringe to inject water, around the extruding pin protected by our new dressing and the absorbent material of the pad will absorb the water and keep the silver pad wet in order to be active.

Following these lengthy (in some cases more than 6 hours in duration) orthopedic surgical procedures, pins remain extruding from the skin. Attending staff are tired and eager to complete the procedure, therefore the use of our system requires only quick flushing of the limb to cleanse the blood, followed by immediate placement of our invented dressing - in comparison to the conventional method where each pin site requires individual cleansing, disinfection and dressing.

In western countries, this type of surgery has been performed since the eighties of the former decade. The major disadvantage of these medical procedures is contamination and nosocomial infections rising out of, and through the exit-sites, as well as the difficulty in keeping the dressing in place for a longer period. The use of adhesive tapes has been known in the art for many years, and despite all of the above mentioned problems, no one has suggested our type of dressing as a solution.

Trials performed, showed that use of our invention will facilitate easy, flat and unwrinkled placement of the adhesive tapes on the skin; as compared with conventional methods of using an adhesive tape that might create wrinkles in the tape when used under a "bridge" and a narrow tight space. It is also important to keep the dressing in place, without falling off, for eight days, even after washing/showering.

The use of only two adhesive tapes, with a pre determined size to fit the pad minimizing the adhesive tape on the skin is a significant advantage. Conventional methods using standard size adhesives cover far too much skin, with all the known disadvantages of adhesive tapes in contact with skin for an extended period. Our experiments revealed that by using pre determined sizes of the pad and adhesive tape, minimal skin covering by the adhesive is achieved.

Current prevailing methods require additional protection (covering sites) when patients with extruding pins are washed or take a shower. Our new invention facilitates washing without any necessity to cover the pin sites, as wetting the silver pad is an advantage, and does not require additional treatment following washing/showering. The complete and flat adhesion of the adhesive tapes around the extruding pin, the pad and the skin, will not allow the dressing to detach or fall off, as compared with water penetration between adhesive and skin if there are wrinkles or air tunnels between the adhesive and the under surfaces of the dressing.

As described above, the ability of "flat adhesion" of the tape cannot be easily achieved by the use of any conventional or previous known method. The easiest and best way is by using our new invention - to completely cover the extruding pin, pad and skin, without wrinkles or air tunnels between adhesive tape and skin surface beneath.

Orthopedic surgeons that used our new invention stated that their findings revealed a definite reduction in infection by more than 1 Log, also saving the use of antibiotics. They also commended the easy, rapid method of use, saving time, and facilitating the bandage to remain in place for seven days even after washing/showering of the patient. Usually, after washing/showering, the conventional adhesive tape falls off, whilst using the new dressing it stayed in place for 7 days.

## Claims

1. An assembly containing at least two adhesive tapes with a U or V shape notch, or a slit that enables them to be placed one opposite the other and the V or U shape or the slit can be placed around an object extruding from a skin exit site minimizing the distance from the base of the U or V or the bottom slit of each adhesive.

2. An assembly as in claim 1 that also contains a pad that can be placed around the extrusion at the skin exit site.

3. An assembly as in claim 2, where instead of at least two adhesive tapes, only one adhesive tape is used.

4. An assembly as in claims 2 and 3, in which the pad is antiseptic.

5. An assembly as in claims 1 to 4, in which the adhesive tape is rectangular, with or without lopped-off edges, round or triangular shaped and the notch in one of the edges of the adhesive tape can be "U" or "V" shaped with the wide part facing outwards, or with a slit vertical to one of the edges that can be straight, with or without a hole at its end, or in a "T" or a cross shape.

6. An assembly as in claims 1 to 5, in which the adhesive tape is porous.

7. An assembly as in claim 6, in which the adhesive on the surface of the non-woven backing of the adhesive tape is in rows, with no adhesive between the rows.

8. An assembly as in claim 7, where the adhesive tapes are pliable and soft in order to keep the dressing on the skin for at least 7 days even after washing or showering.

9. A method of placing an antiseptic pad around an object extruding from a skin exit site; an adhesive tape with a "U" or "V" shaped notch with the wide edge facing outwards, or slit cut vertically to one of its edges, is adhered to affix the pad to the skin so that the extruded member from the exit site reaches the bottom of the "U" or the "V" or the slit as closely as possible; afterwards, another adhesive tape with a "U" or "V" shape or slit at one of its edges, is adhered to affix the pad to the skin so that the extruding member from the exit site reaches the bottom of the "U" or the "V" or the slit as closely as possible.

10. A method of placing an antiseptic pad around an object extruding from a skin exit site; an adhesive tape with a "U" or "V" shaped notch with the wide edge facing outwards, or slit cut vertically to one of its edges, is adhered to affix the pad to the skin so that the extruded member from the exit site reaches the bottom of the "U" or the "V" or the slit as closely as possible.
